# EUROPÄISCHE PATENTSCHRIFT

(11) **EP 2 695 631 B1**
(45) Veröffentlichungstag und Bekanntmachung des Hinweises auf die Patenterteilung: **25.08.2021**
(21) Anmeldenummer: 13178010.8
(22) Anmeldetag: 25.07.2013
(51) Int. Cl.: A61M 21/00

(54) **Therapiegerät für die biomechanische Stimulation in der Human- und Veterinärmedizin**
Therapeutic device for biomechanical stimulation in human and veterinary medicine
Appareil de thérapie pour la stimulation biomécanique en médecine humaine et vétérinaire

(30) Priorität: 06.08.2012 AT 8682012
(43) Veröffentlichungstag der Anmeldung: 12.02.2014
(73) Patentinhaber: Galler, Joachim, 8572 Bärnbach (AT)
(72) Erfinder: Galler, Joachim, 8572 Bärnbach (AT)
(74) Vertreter: Margotti, Herwig Franz

(56) Entgegenhaltungen:
- US-A- 4 605 975
- US-A- 5 324 225
- US-A1- 2003 147 546
- US-A1- 2004 130 449
- US-A1- 2004 176 138
- US-A1- 2009 083 908
- US-A1- 2009 156 089
- US-A1- 2011 061 661

## Beschreibung

Die Erfindung betrifft ein Therapiegerät für die biomechanische Stimulation in der Human- und Veterinärmedizin, mit einem Schallwandler zur direkten Wiedergabe von Schnurrfrequenz-Signalen einer Katze in Form von Vibrationen auf einen menschlichen oder tierischen Körper.

Die Druckschrift US 2009/156089 A1 offenbart eine künstliche Katze mit einer Vorrichtung, welche Laute und Vibrationen eines realen Tieres imitieren kann.

US 5 324 225 A offenbart ebenfalls eine künstliche Katze mit einer Vorrichtung zur Lauterzeugung, sowie einem Motor zur Bewegung des Schwanzes.

US 2003/147546 A1 offenbart einen Polster mit einem integirerten Modul zur erzeugung von Schnurrlauten.

US 2011/061661 A1 offenbart ebenfalls einen Polster zur Erzeugung von Vibrationen und Schnurrlauten

US 2004/130449 A1 offenbart eine elektronisches Gerät zur Beruhigung eines Kleinkinds mit einer Funkeinheit, welche dazu ausgebildet ist automatisch aktiviert zu werden, wenn das Kleinkind zu weinen beginnt, und in Reaktion darauf aufgezeichnete Geräusche oder Stimmen abzuspielen.

US 2009/083908 A1 offenbart einen antibakteriellen, anti-allergenen Polster.

Aufgrund von Forschungsergebnissen hat man herausgefunden, dass nicht jedes Schnurrfrequenz-Signal für die Therapieanwendung geeignet ist.

Es ist daher die Aufgabe der Erfindung, ein für die Therapieanwendung geeignetes Gerät für die biomechanische Stimulation in der Human- und Veterinärmedizin anzugeben, das einen Schallwandler zur direkten Wiedergabe von Schnurrfrequenz-Signalen einer Katze in Form von Vibrationen auf einen menschlichen oder tierischen Körper umfasst.

Erfindungsgemäß weist das Therapiegerät einen Speicher für die mittels eines Körperschallmikrofons an einer Katze aufgenommenen Schnurrfrequenz-Signale, sowie einen Verstärker zur Verstärkung der im Speicher gespeicherten Schnurrfrequenz-Signale und zur Weiterleitung der verstärkten Signale an den Schallwandler auf.

Es sind weiters ein am Kehlkopf einer Katze anordenbares Körperschallmikrofon und eine Aufzeichnungsvorrichtung, die die vom Körperschallmikrofon aufgenommenen Schnurrfrequenz-Signale im Speicher abspeichert, vorgesehen, wobei die Aufzeichnungsvorrichtung vorzugsweise als Computer ausgebildet ist. Somit kann das Körperschallmikrophon an einer Katze angeordnet werden, die die geeignete Frequenz für die Therapieanwendung liefert, und die Schnurrfrequenz dieser Katze wird als Körperschall vom Mikrophon abgenommen und, vorzugsweise elektronisch, für die Wiedergabe gespeichert.

Versuche des Erfinders haben gezeigt, dass die Schnurrfrequenz-Signale vorteilhaft mittels eines Filters auf eine Bandbreite zwischen 0 und 700 Hz begrenzt werden können.

In einer bevorzugten Ausführungsform des erfindungsgemäßen Therapiegeräts umfasst der Schallwandler einen Permanentmagneten, eine elektrische Spule, die von den verstärkten Schnurrfrequenz-Signalen durchflossen wird, wodurch der Permanentmagnet und die elektrische Spule in Schwingungsbewegung zueinander versetzt werden, und einen Schwingungsträger, wie z.B. eine Kunststoff- oder Faserplatte, der am Permanentmagneten oder an der elektrischen Spule befestigt ist. In einer Fortbildung dieser Ausführungsform weist das Therapiegerät ein Gehäuse auf, das mit dem beweglichen Teil des Schwingungsträgers über einen Luftspalt als magnetischen Rückschluss verbunden ist.

Damit die Schnurrfrequenz-Signale optimal übertragen werden, ist es zweckmäßig, wenn der Schallwandler in einen Kunststoff oder Schaumstoff eingebettet ist, der die Schnurrfrequenz-Signale nicht absorbiert.

Weiters ist es bevorzugt, wenn der Speicher ein Datenträger in Form einer SD-Card, eines SD-Sticks oder eines Chip ist und die Schnurr-Frequenz-Signale als Datei, z.B. als mp3-Datei, darauf gespeichert sind.

Aus hygienischen Gründen wird vorgeschlagen, dass das Therapiegerät durch eine äußere Schutzhülle aus desinfizierbarem Kunststoff geschützt wird.

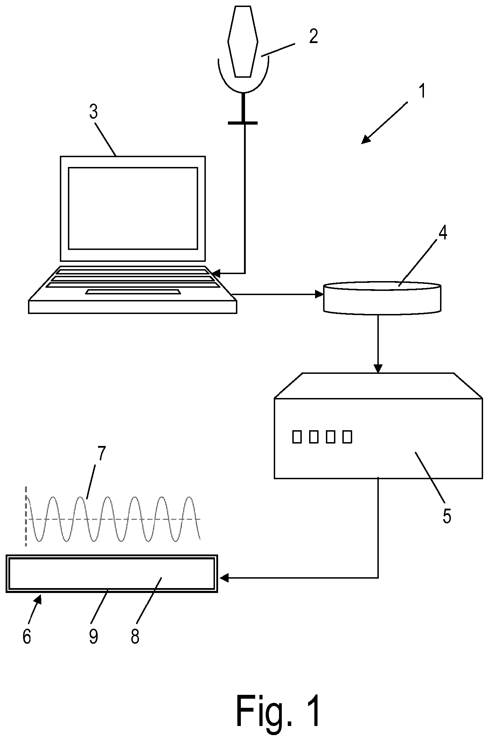

Eine Ausführungsform des erfindungsgemäßen Therapiegeräts ist in der einzigen Figur 1 schematisch dargestellt.

Das Therapiegerät 1 für die biomechanische Stimulation in der Human- und Veterinärmedizin umfasst eine Aufzeichnungseinheit mit einem Körperschallmikrophon 2, das Schnurrfrequenz-Signalen einer Katze aufnimmt und in Form von elektrischen Schnurrfrequenz-Signalen an eine Filter- und Verarbeitungseinheit 3 in Form eines Computers überträgt, der die empfangenen Schnurrfrequenz-Signale aufzeichnet, herausfiltert und abspeichert, genauer gesagt, der den Teil des Schnurrfrequenzspektrums zwischen 0-700 Hz in eine fertige Datei, beispielsweise in ein mp3- File umwandelt und in einem Speicher 4, z.B. eine SD-Card, einen SD-Stick oder einen Chip speichert. Die Frequenzen werden entsprechend den Zellfrequenzen bearbeitet. Oberwellen und Störimpulse werden herausgefiltert. Gegebenenfalls wird in dem Speicher 4 auch Software gespeichert. Eine Wiedergabeeinheit des Therapiegeräts 1 umfasst einen Verstärker 5 zur Verstärkung der im Speicher 4 gespeicherten Schnurrfrequenz-Signale und zur Weiterleitung der verstärkten Signale an einen in einem Therapiekissen 6 enthaltenen Schallwandler 8 zur direkten Wiedergabe der Schnurrfrequenz-Signale 7 in Form von Vibrationen auf einen menschlichen oder tierischen Körper.

Der Verstärker 5 ist ein lüfterloser Verstärker im Arbeitsbereich zwischen 0-700 Hz, der die im Speicher 4 als mp3 File gespeicherten Katzenschnurr-Signalfrequenzen verstärkt und dem Schallwandler im Therapiekissen 6 zuführt. Danach werden diese Frequenzen vom Schallwandler 8 mittels einer Spule in eine Vibration zurückgewandelt. Dabei können Ausgangsleistungen zwischen 0-15 W/ bei 4 bzw. 8 Ohm auftreten.

Das Schnurrkissen bzw. Therapiekissen 6 kann in unterschiedlichsten Größen oder Ausführungen gefertigt werden. Idealerweise hat man sich für die Anwendung beim Menschen auf eine Größe von ca.20x20x8cm festgelegt. Man kann aber auch das System beispielsweise für Autositze, Flugzeugsitze, Liegen oder Matratzen adaptieren.

Im Therapiekissen 6 ist ein Schallwandler 8 mit Permanentmagnet und Spule eingebaut. Auf einem Schwingungsträger wie z.B. einer Kunststoffplatte, oder Faserplatte wird der oben beschriebene Dauermagnet mit magnetischem Rückschluss oder die Spule befestigt. Der jeweils andere Bauteil kann frei schwingen.

Das Gehäuse selbst kann aus den unterschiedlichsten Materialien wie Kunststoff, Metall oder Holz bestehen. Das Gehäuse ist mit dem beweglichen Teil des Schwingungsträgers über den Luftspalt als magnetischen Rückschluss verbunden.

Die elektrischen Werte der Spule und die geometrischen Abmessungen des Therapiekissens 6 ergeben sich je nach den Anforderungen und der benötigten Therapieleistung.

Das elektromechanische System wird in einen Kunststoff bzw. Schaumstoff gebettet. Der Kunststoff muss so gewählt werden, sodass die Schnurrfrequenzen nicht absorbiert werden und die Frequenzinformationen für die menschlichen Zellen erhalten bleiben.

Eine Schutzhülle 9 aus beispielsweise Kunststoffleder, geeignet für die Reinigung und Desinfektion, komplettiert das Schnurrkissen.

## Patentansprüche

1. Therapiegerät (1) für die biomechanische Stimulation in der Human- und Veterinärmedizin, mit einem Schallwandler (8) zur direkten Wiedergabe von Schnurrfrequenz-Signalen (7) einer Katze in Form von Vibrationen auf einen menschlichen oder tierischen Körper, **gekennzeichnet durch** einen Speicher (4), ein am Kehlkopf einer Katze anordenbares Körperschallmikrofon (2) und eine Aufzeichnungsvorrichtung (3), die die vom Körperschallmikrofon (2) aufgenommenen Schnurrfrequenz-Signale im Speicher (4) abspeichert, wobei die Aufzeichnungsvorrichtung (3) vorzugsweise als Computer ausgebildet ist, sowie einen Verstärker (5) zur Verstärkung der im Speicher (4) gespeicherten Schnurrfrequenz-Signale und zur Weiterleitung der verstärkten Signale an den Schallwandler (8).

2. Therapiegerät nach Anspruch 1, **gekennzeichnet durch** einen Filter, der die Schnurrfrequenz-Signale auf eine Bandbreite zwischen 0 und 700 Hz begrenzt.

3. Therapiegerät nach einem der vorhergehenden Ansprüche, **dadurch gekennzeichnet, dass** der Schallwandler (8) einen Permanentmagneten, eine elektrische Spule, die von den verstärkten Schnurrfrequenz-Signalen durchflossen wird, wodurch der Permanentmagnet und die elektrische Spule in Schwingungsbewegung zueinander versetzt werden, und einen Schwingungsträger, wie z.B. eine Kunststoff- oder Faserplatte, der am Permanentmagneten oder an der elektrischen Spule befestigt ist, umfasst.

4. Therapiegerät nach Anspruch 3, **gekennzeichnet durch** ein Gehäuse, das mit dem beweglichen Teil des Schwingungsträgers über einen Luftspalt als magnetischen Rückschluss verbunden ist.

5. Therapiegerät nach einem der vorhergehenden Ansprüchen, **dadurch gekennzeichnet, dass** der Schallwandler (8) in einen Kunststoff oder Schaumstoff eingebettet ist, der die Schnurrfrequenz-Signale nicht absorbiert.

6. Therapiegerät nach einem der vorhergehenden Ansprüchen, **dadurch gekennzeichnet, dass** der Speicher (4) ein Datenträger in Form einer SD-Card, eines SD-Sticks oder eines Chip ist und die Schnurr-Frequenz-Signale (7) als Datei, z.B. als mp3-Datei, darauf gespeichert sind.

7. Therapiegerät nach einem der vorhergehenden Ansprüchen, **gekennzeichnet durch** eine äußere Schutzhülle (9) aus desinfizierbarem Kunststoff.

## Claims

1. A therapeutic device (1) for biomechanical stimulation in human and veterinary medicine having a sound transducer (8) for direct playback of purr frequency signals (7) of a cat in the form of vibrations on a human or animal body, **characterized by** a memory (4), a structure-borne sound microphone (2) placeable at the larynx of a cat, and a recording device (3) that stores the purr frequency signals recorded by the structure-borne sound microphone (2) in the memory (4), wherein the recording device (3) is preferably designed as a computer, as well as an amplifier (5) for amplifying the purr frequency signals stored in the memory (4) and transmitting the amplified signals to the sound transducer (8).

2. The therapeutic device according to claim 1, **characterized by** a filter that limits the purr frequency signals to a bandwidth between 0 and 700 Hz.

3. The therapeutic device according to any one of the preceding claims, **characterized in that** the sound transducer (8) comprises a permanent magnet, an electric coil through which the amplified purr frequency signals flow, whereby the permanent magnet and the electric coil are set into oscillatory motion to one another, and an oscillation carrier, such as a plastic or fiber board, which is attached to the permanent magnet or to the electric coil.

4. The therapeutic device according to claim 3, **characterized by** a housing which is connected to the movable part of the oscillation carrier via an air gap as magnetic return path.

5. The therapeutic device according to any one of the preceding claims, **characterized in that** the sound transducer (8) is embedded in a plastic or foam material that does not absorb the purr frequency signals.

6. The therapeutic device according to any one of the preceding claims, **characterized in that** the memory (4) is a data carrier in the form of an SD card, an SD stick or a chip and the purr frequency signals (7) are stored thereon as a file, e.g. as an mp3 file.

7. The therapeutic device according to any one of the preceding claims, **characterized by** an outer protective cover (9) made of disinfectable plastic.

## Revendications

1. Appareil de thérapie (1) pour la stimulation biomécanique en médecine humaine et vétérinaire, comprenant un transducteur acoustique (8) pour la reproduction directe de signaux de fréquence de ronronnement (7) d'un chat sous forme de vibrations sur un corps humain ou animal, **caractérisé par** une mémoire (4), un microphone de bruit solidien (2) pouvant être disposé sur le larynx d'un chat et un dispositif d'enregistrement (3) pour mémoriser les signaux de fréquence de ronronnement reçus par le microphone de bruit solidien (2) dans la mémoire (4), dans lequel le dispositif d'enregistrement (3) est réalisé de préférence sous la forme d'un ordinateur, ainsi que par un amplificateur (5) pour amplifier les signaux de fréquence de ronronnement mémorisés dans la mémoire (4) et pour transmettre les signaux amplifiés au transducteur acoustique (8).

2. Appareil de thérapie selon la revendication 1, **caractérisé par** un filtre qui limite les signaux de fréquence de ronronnement à une bande passante qui est comprise entre 0 Hz et 700 Hz.

3. Appareil de thérapie selon l'une quelconque des revendications précédentes, **caractérisé en ce que** le transducteur acoustique (8) comprend un aimant permanent, une bobine électrique qui est traversée par les signaux de fréquence de ronronnement amplifiés, ce qui a pour effet que l'aimant permanent et la bobine électrique sont mis en mouvement de vibration l'un par rapport à l'autre, et un support de vibrations, tel que par exemple un panneau de matière plastique ou de fibres, qui est fixé sur l'aimant permanent ou sur la bobine électrique.

4. Appareil de thérapie selon la revendication 3, **caractérisé par** un boîtier qui est relié à la partie mobile du support de vibrations par l'intermédiaire d'un entrefer qui fait office de culasse magnétique.

5. Appareil de thérapie selon l'une quelconque des revendications précédentes, **caractérisé en ce que** le transducteur acoustique (8) est intégré dans une matière plastique ou de mousse qui n'absorbe pas les signaux de fréquence de ronronnement.

6. Appareil de thérapie selon l'une quelconque des revendications précédentes, **caractérisé en ce que** la mémoire (4) est un support de données qui se présente sous la forme d'une carte SD, d'un stick SD ou d'une puce et les signaux de fréquence de ronronnement (7) sont mémorisés sur celui-ci en tant que fichier, par exemple en tant que fichier mp3.

7. Appareil de thérapie selon l'une quelconque des revendications précédentes, **caractérisé par** une enveloppe protectrice extérieure (9) en matière plastique qui peut être désinfectée.
